# EUROPÄISCHE PATENTSCHRIFT

(11) **EP 2 934 355 B1**
(45) Veröffentlichungstag und Bekanntmachung des Hinweises auf die Patenterteilung: **21.04.2021**
(21) Anmeldenummer: 13815722.7
(22) Anmeldetag: 18.12.2013
(51) Int. Cl.: A61B 17/72

(54) **VERRIEGELBARER MARKNAGEL MIT FÜHRUNGSDRAHTDURCHLÄSSEN**
LOCKABLE INTRAMEDULLARY NAIL HAVING GUIDE WIRE HOLES
CLOU INTRAMEDULLAIRE À VERROUILLAGE COMPRENANT DES TROUS POUR UN FIL DE GUIDAGE

(30) Priorität: 18.12.2012 DE 102012024688
(43) Veröffentlichungstag der Anmeldung: 28.10.2015
(73) Patentinhaber: OT Medizintechnik GmbH, 80639 München (DE)
(72) Erfinder: SCHREIBER, Ulrich, 80639 München (DE)
(74) Vertreter: Bobbert & Partner Patentanwälte PartmbB
(86) Internationale Anmeldenummer: PCT/EP2013/077207
(87) Internationale Veröffentlichungsnummer: WO 2014/096079

(56) Entgegenhaltungen:
- EP-A2- 2 335 627
- WO-A1-2007/093855
- WO-A1-2011/002753
- WO-A2-2005/094707
- WO-A2-2008/141805
- US-A1- 2012 197 255

## Beschreibung

Die Erfindung bezieht sich auf einen Marknagel und eine Vorrichtung zum Fixieren des Marknagels in einem Röhrenknochen gemäß dem Oberbegriff des unabhängigen Patentanspruchs.

Marknägel und speziell verriegelbare Marknägel sind bekannte Hilfsmittel zur Versorgung von Brüchen langer Röhrenknochen, die in den Markraum des lädierten Knochens eingebracht werden, um die Knochenläsion mechanisch zu überbrücken. Bei den so genannten Verriegelungsmarknägeln dienen Verriegelungsschrauben, -stifte oder allgemein -einrichtungen, welche Knochen und Verriegelungsnagel überbrückend und/oder verbindend, insbesondere quer durch oder in den Knochen und den Verriegelungsnagel oder Abschnitte hiervon, eingebracht sind, dazu, die Verbindung zwischen Knochen und Verriegelungsnagel gegen Verschiebung, insbesondere des Verriegelungsnagels, in Richtung der Knochenachse sowie gegen Rotation des Marknagels um dessen Längsachse zu sichern WO2005094707 und WO2008141805 beschreiben einen Marknagel zum Einbringen in einen Röhrenknochen, mit einem Schaft mit einer Längsachse, wobei der Schaft wenigstens eine Durchgangsöffnung zum Einbringen wenigstens einer Verriegelungseinrichtung in ein Inneres des Marknagels aufweist wobei der Marknagel wenigstens eine Aufnahmeeinrichtung zum Aufnehmen wenigstens eines Abschnitts der Verriegelungseinrichtung aufweist; wobei die wenigstens eine Aufnahmeeinrichtung eine Hülse in Form einer Walze oder eine kugelförmige oder elliptische Hülse ist, die in wenigstens einer ihrer Raumachsen in Bezug zur Längsachse des Schafts bewegbar oder ausrichtbar ist wobei der Marknagel wenigstens eine Einrichtung zum Unterbinden der Ausrichtbarkeit aufweist; und wobei die wenigstens eine Aufnahmeeinrichtung eine Öffnung oder Bohrung zur Aufnahme der wenigstens einen Verriegelungseinrichtung aufweist.

Aufgabe der vorliegenden Erfindung ist es, einen weiteren Marknagel, insbesondere einen Verriegelungsmarknagel (welcher im Folgenden auch als Marknagel bezeichnet ist) anzugeben. Die erfindungsgemäße Aufgabe wird durch einen Marknagel oder Verriegelungsmarknagel mit den Merkmalen des unabhängigen Anspruchs gelöst.

Demzufolge wird erfindungsgemäß ein Marknagel zum Einbringen in einen Röhrenknochen vorgeschlagen, der einen Schaft mit einer Längsachse wie in Anspruch 1 angegeben aufweist. Der Schaft weist wenigstens eine Durchgangsöffnung zum Einbringen wenigstens einer Verriegelungseinrichtung, insbesondere einer Verriegelungsschraube oder eines Verriegelungsstifts in ein Inneres des Marknagels und/oder zum Hindurchführen durch den Marknagel auf.

Der Marknagel weist wenigstens eine Aufnahmeeinrichtung zum Aufnehmen wenigstens eines Abschnitts der Verriegelungseinrichtung auf.

Die wenigstens eine Aufnahmeeinrichtung ist in wenigstens einer ihrer Raumachsen in Bezug zur Längsachse des Schafts bewegbar oder ausrichtbar. Sie ist insbesondere drehbar oder verschwenkbar, am Marknagel, innerhalb des Schafts und/oder in der Durchgangsöffnung angeordnet.

Der Marknagel weist wenigstens eine Einrichtung, insbesondere eine Arretiereinrichtung und/oder eine Ausgestaltung von Verriegelungseinrichtung und/oder Aufnahmeeinrichtung, zum Unterbinden der Ausrichtbarkeit der wenigstens einen Aufnahmeeinrichtung in Bezug zur Längsachse und/oder zu einer hierzu senkrechten Querachse auf.

Die wenigstens eine Aufnahmeeinrichtung weist wenigstens eine durch sie hindurchreichende Führungsdrahtöffnung zum Aufnehmen, Hindurchführen oder Umgeben eines Führungsdrahts auf. Die wenigstens eine Führungsdrahtöffnung kann synonym auch als Führungsdrahtdurchlass oder als Führungsdrahtführung bezeichnet werden.

Unter einem Führungsdraht wird in gewissen erfindungsgemäßen Ausführungsformen ein Draht (oder eine hierfür ebenfalls einsetzbare und/oder in der Praxis bereits eingesetzte Struktur) verstanden, welcher vor dem Einbringen eines orthopädischen oder chirurgischen Instruments und/oder eines Implantats in das Gewebe, beispielsweise in den Knochen, eingebracht wird. Der Führungsdraht ist dabei vorgesehen, um an ihm entlang, ihn umgebend, ihn einschließend, mit ihm verbunden und/oder durch ihn geführt in einem späteren, zweiten Arbeitsschritt ein Instrument oder ein Implantat in jenes Gewebe einzubringen, in welches der Führungsdraht in einem früheren, ersten Arbeitsschritt bereits eingebracht wurde. Der Führungsdraht dient in diesen Ausführungsformen somit als *path finder,* Wegbereiter oder Leitstruktur für das Instrument oder das Implantat, indem er dessen ortsgerechte Einbringung in dasselbe Gebewebe vorbereitet und/oder erleichtert.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll beispielhafte offenbarte Ausführungsformen erläutern.

Beispielhafte Ausführungsformen der vorliegenden Offenbarung können eines oder mehrere der im Folgenden genannten Merkmale, einzeln oder in beliebiger Kombination, aufweisen.

Weitere beispielhafte Ausgestaltungen der Erfindung sind auch jeweils Gegenstand der Unteransprüche.

So ist die wenigstens eine Führungsdrahtöffnung des erfindungsgemäßen Marknagels in manchen beispielhaften Ausführungsformen verschieden von einer Durchgangsöffnung der Aufnahmeeinrichtung, durch welche die Verriegelungseinrichtung durch die Aufnahmeeinrichtung hindurchtritt.

In gewissen offenbarten Ausführungsformen ist die Führungsdrahtöffnung zu klein im Durchmesser, um eine Verriegelungsschraube durch sie hindurch zu führen.

In manchen offenbarten Ausführungsformen ist der Marknagel ein Arthrodesenagel, beispielsweise zum Versteifen von Gelenken.

In einigen beispielhaften Ausführungsformen des erfindungsgemäßen Marknagels verläuft die wenigstens eine Führungsdrahtöffnung in manchen ihrer Abschnitte durch die Durchgangsöffnung der Aufnahmeeinrichtung hindurch, in anderen Abschnitten hingegen nicht.

In bestimmten beispielhaften Ausführungsformen des erfindungsgemäßen Marknagels weist die wenigstens eine Führungsdrahtöffnung einen Durchmesser oder eine Breite in einem Bereich zwischen 1 bis 5 mm, vorzugsweise zwischen 2 und 4 mm, vorzugsweise 3 mm auf. In gewissen beispielhaften offenbarten Ausführungsformen weist die Führungsdrahtöffnung einen Durchmesser oder eine Breite von maximal 3 mm auf. Dies erlaubt vorteilhaft eine ausreichende Dicke und Festigkeit der die Führungsdrahtöffnung umgebenden Strukturen.

In einigen beispielhaften Ausführungsformen des erfindungsgemäßen Marknagels verläuft die wenigstens eine Führungsdrahtöffnung nicht nur durch die eine oder die mehreren Aufnahmeeinrichtung(en), sondern kann ferner durch optional vorgesehene Zwischenkörper, welche zwischen benachbarten Aufnahmeeinrichtungen optional vorgesehen sein können, verlaufen. Entsprechende Öffnungen oder Durchlässe können auch hierin vorgesehen sein.

Erfindungsgemäße Ausführungsformen können einen oder mehrer der hierin genannten Vorteile aufweisen. Zu ihnen zählen die im Folgenden genannten.

Das Vorsehen der Führungsdrahtöffnung erlaubt es vorteilhaft, den erfindungsgemäßen Marknagel entlang eines durch den Marknagel verlaufenden Führungsdrahts einzubringen. Der Marknagel wird dabei erst dann in den Knochen eingetrieben, wenn seine künftige Lage und sein Einbringweg anhand des zuvor eingebrachten Führungsdrahts sondiert und festgelegt sowie ggf. auch - beispielsweise röntgentechnisch - überprüft sind. Auf diese Weise darf der Chirurg vor dem Einbringen des Marknagels mit größerer Sicherheit davon ausgehen, dass der Marknagel so zum Liegen kommen wird, wie gewünscht.

Ein weiterer Vorteil von Führungsdrahtöffnungen kann darin bestehen, dass mittels eines hierin eingeführten Führungsdrahts die Stellung der Aufnahmeeinrichtung während des mehr oder weniger gewaltsamen Einschlagens des Marknagels in den Knochen zuverlässig gewahrt wird. Der Führungsdraht verhindert ein unbeabsichtigtes Verdrehen der einen oder der mehreren Aufnahmeeinrichtungen, welche sich durch Erschütterungen während des Einbringens usw. ergeben können. Aufgrund der mittels des Führungsdrahts gewahrten Stellung der Aufnahmeneinrichtung bezogen auf den Marknagel oder innerhalb des Marknagels kann sichergestellt werden, dass Öffnungen der einzelnen Aufnahmeeinrichtungen, durch welche nach Einbringen des Marknagels der oder die Verriegelungsstift(e) gesteckt werden, auch nach dem Einbringen des Marknagels in den Knochen mit jenen Öffnungen des Marknagels, durch welche die Verriegelungsstifte ebenfalls gesteckt werden müssen, noch fluchten (oder gar erstmals fluchten). Dies erleichtert das Einbringen der Verriegelungsschrauben in Knochen und Marknagel merklich.

In einigen beispielhaften Ausführungsformen des erfindungsgemäßen Marknagels weist dieser auch jenseits der Aufnahmeeinrichtung(en) wenigstens eine Öffnung oder einen Führungsdrahtdurchlass für einen Führungsdraht auf, welche den Führungsdraht innerhalb des Marknagels zumindest im Bereich der Öffnung/des Durchlasses führt. Der Führungsdraht kann hierzu vergleichsweise eng durch die Öffnung geführt werden oder diese nur geringfügig weiter als der Durchmesser des Führungsdrahts sein. Dies erlaubt ein wirkungsvolles Führen des Führungsdrahts im Bereich der Öffnung mittels des Führungsdrahtdurchlasses und relativ zum Marknagel.

In gewissen beispielhaften Ausführungsformen des erfindungsgemäßen Marknagels ist/sind die Führungsdrahtöffnung(en) der Aufnahmeeinrichtung(en) nicht in einem Symmetriemittelpunkt der Aufnahmeeinrichtung(en) vorgesehen. Auf diese Weise ist eine wie oben beschriebene Ausrichtung der Aufnahmeeinrichtung(en) selbst dann vorteilhaft möglich, wenn die Aufnahmeeinrichtung beispielsweise eine Kugel ist.

In einigen beispielhaften Ausführungsformen des erfindungsgemäßen Marknagels sind die Durchgangsöffnungen für die Verriegelungsschraube(n) Durchbrüche, in deren Bereich die Mantelfläche des Marknagels durchbrochen oder gegenüber einem Äußeren des Marknagels geöffnet ist.

In manchen beispielhaften Ausführungsformen des erfindungsgemäßen Marknagels ist die wenigstens eine Aufnahmeeinrichtung kein integraler Bestandteil des Marknagels sondern ein hiervon separates, ggf. auswechselbares Bauteil.

In einigen beispielhaften Ausführungsformen des erfindungsgemäßen Marknagels ist die Aufnahmeeinrichtung im Bereich zumindest einer Öffnung des Marknagels als zumindest in einer Raumrichtung - bspw. in einer Richtung senkrecht zu der Längsachse des Knochen- oder Marknagelschaftes- und/ oder in einer Ebene, insbesondere einer die zuvor genannte Richtung enthaltenden Ebene - ausrichtbar, oder bewegbar oder verschiebbar oder verschwenkbar in dem Schaft und/ oder am Schaft angeordnet und/oder gelagert.

Die Aufnahmeeinrichtung weist in einigen beispielhaften Ausführungsformen des erfindungsgemäßen Marknagels eine Öffnung oder Bohrung zur Aufnahme einer Verriegelungsschraube oder mehrere solcher oder anderer Verriegelungseinrichtungen auf. Nach Einsetzen der Verriegelungsschraube(n) in den Schaft kann diese Aufnahmeeinrichtung wiederum verriegelt bzw. blockiert bzw. arretiert werden.

In manchen beispielhaften Ausführungsformen des erfindungsgemäßen Marknagels sind eine, mehrere oder alle der vorhandenen Aufnahmeeinrichtungen als Hülsen ausgestaltet oder weisen wenigstens eine solche auf.

Es gilt hierin, dass wenn im Folgenden daher von einer Hülse der Rede ist, dieser Begriff nicht hierauf beschränkend zu verstehen ist. Das für die Hülse jeweils Gesagte trifft in anderen beispielhaften offenbarten Ausführungsformen auch auf die Aufnahmeeinrichtung im weitesten Sinne zu, sofern der Fachmann hierin keinen Widerspruch erkennt.

In einigen beispielhaften Ausführungsformen des erfindungsgemäßen Marknagels kann die Verschwenkbarkeit der Hülse und ihre Nicht-Verschwenkbarkeit nach Verriegelung oder Blockung an einer zugelassenen oder unterbundenen Bewegung, insbesondere Neigbarkeit oder Drehbarkeit, einer in Bezug auf die Hülse festlegbaren virtuellen Achse oder Geraden bestimmt oder festgemacht werden. Diese Achse kann eine Längsachse oder eine Symmetrieachse der Öffnung oder Bohrung der Hülse sein. In manchen beispielhaften Ausführungsformen des erfindungsgemäßen Marknagels kann die Aufnahmeeinrichtung eine Einrichtung mit wenigstens einer vorgefertigten Durchgangsöffnung für die Verriegelungsschraube sein. Unter den Begriff "Hülse" oder Aufnahmeeinrichtung fällt neben der vom Fachmann unter Hülse oder Aufnahmeeinrichtung verstandenen Struktur erfindungsgemäß auch jede Einrichtung, welche keine vorgefertigte Durchgangsöffnung aufweist, sondern bei welcher ein Durchlass für die Verriegelungsschraube beim Einbringen der Verriegelungsschraube und ggf. auch erst durch das Einbringen entsteht.

In einigen beispielhaften Ausführungsformen des erfindungsgemäßen Marknagels umfasst "Hülse" oder Aufnahmeeinrichtung somit auch eine Masse, insbesondere eine aushärtbare Masse wie Knochenzement, welche zu einem frühen ersten Zeitpunkt ein Ausrichten des oder der durch die hindurch geführte(n) Verriegelungsschraube(n) erlaubt, nicht mehr aber zu einem späteren zweiten Zeitpunkt. Auch eine Arretierung mittels eines Materials, welches bei unterschiedlichen Umgebungstemperaturen unterschiedliche Formen einnimmt und sich bei Wärme ausdehnen oder zusammenziehen kann, wird, wenn der Arretiereffekt durch diese Materialeigenschaft bewirkt oder begünstigt wird, in manchen beispielhaften Ausführungsformen unter der Aufnahmeeinrichtung oder Hülse verstanden.

Die Hülse ist in manchen beispielhaften Ausführungsformen des erfindungsgemäßen Marknagels eine Walze mit einer Walzenachse, welche bspw. senkrecht zu der Längsachse des Schafts des Verriegelungsnagels steht. In anderen beispielhaften Ausführungsformen des erfindungsgemäßen Marknagels ist sie vorteilhaft kugelförmig oder von elliptischem oder elliptoidem Durchmesser, so dass sie in mehreren, vorzugsweise in allen Raumachsen frei schwenkbar im Schaft gelagert ist.

Die Aufnahmeeinrichtung ist in einigen beispielhaften Ausführungsformen des erfindungsgemäßen Marknagels aus mehreren Teilen und/ oder mehreren Materialien zusammengesetzt (Composit). Beispielsweise kann ein Kunststoffring als Teil der Aufnahmeeinrichtung zum Einsatz kommen. Er kann ein ungewolltes Herausdrehen der Verriegelungsschraube(n) vorteilhaft verhindern.

In einigen beispielhaften Ausführungsformen des erfindungsgemäßen Marknagels weist dieser wenigstens oder genau eine, insbesondere zum Schaft des Marknagels koaxial und/oder im Wesentlichen parallel zur Längsachse des Marknagels angeordnete Klemmeinrichtung, beispielsweise eine Klemmschraube zum Unterbinden der Ausrichtbarkeit der wenigstens einen oder von mehreren oder allen

Aufnahmeeinrichtungen und/oder zur Fixierung von deren Lage auf.

Unter der "Bohrung", welche in der Aufnahmeeinrichtung vorgesehen ist, wird erfindungsgemäß eine Bohrung verstanden, wie sie dem Fachmann bekannt ist. Zusätzlich kann auch eine Durchgangsöffnung zur Aufnahme der Verriegelungsschraube oder eines Abschnitts hiervon vorgesehen sein, welche der Fachmann nicht als eine Bohrung ansieht. Eine solche Durchgangsöffnung kann bspw. gegossen oder bei einem Gießvorgang (mit-)entstanden sein. Beim Gießen muss - anders als beim Erzeugen einer Bohrung kein Hülsenmaterial abgetragen oder beseitigt werden. Eine solche nicht gebohrte Durchgangsöffnung kann ferner durch Zusammenbringen mehrerer Teil-Aufnahmeeinrichtungen mit entsprechenden Aussparungen zustande kommen.

Unter einer Verriegelung wird in manchen beispielhaften Ausführungsformen des erfindungsgemäßen Marknagels ein Verhindern einer Bewegung - z.B. des Marknagels - in einer Längsachse hiervon oder um seine Längsachse herum nach Abschluss dessen Einbringen in den Knochen verstanden. Es wird im Zusammenhang mit der vorliegenden Erfindung jedoch auch ein Fixieren von Frakturfragmenten oder anderen Knochenabschnitten verstanden. Unter Fixieren wird auch der Versuch verstanden, den Abstand zwischen Knochenabschnitten oder -fragmenten zu bewahren. Ein Sintern, insbesondere ein ärztlich gewolltes Sintern, der Knochenabschnitte, bei welchem sich der Abstand trotz Verriegelung in begrenztem Umfang verändern darf, ist hierbei dem Fixieren nicht abträglich. Daher kann eine Verriegelungsschraube oder -einrichtung im Sinne der vorliegenden Erfindung sowohl eine Einrichtung zum Fixieren des Marknagels im Knochen, als auch eine Einrichtung zum Sichern von wenigstens zwei Knochen oder -abschnitten oder -fragmenten relativ zueinander sein. Mit einer Konstruktion eines Marknagels gemäß der Erfindung können beim Einsetzen einer Verriegelungsschraube in den Marknagel in manchen beispielhaften Ausführungsformen geringe Fehlstellungen der Verriegelungsschraube quasi automatisch ausgeglichen werden. Zudem hat der Operateur, insbesondere bei einer kugelförmigen Hülse, die in drei Freiheitsgraden ausrichtbar ist, die Möglichkeit, noch während der Operation den Winkel der den Marknagel durchdringenden Verriegelungsschraube(n) bspw. zum Schaft und/oder die Ausrichtung der Verriegelungsschraube(n) im Raum variieren zu können. Hierdurch hat der Operateur eine Möglichkeit, die Frakturfragmente des Knochens oder des Gelenkes präzise zu repositionieren beziehungsweise anatomisch korrekt zu adaptieren, bzw. zu komprimieren.

Die Verriegelung der Aufnahmeeinrichtung mit der Verriegelungsschraube, beziehungsweise die Lagefixierung oder Blockierung der Aufnahmeeinrichtung, kann auf mehrere Arten erfolgen. Die Aufnahmeeinrichtung, die nur bevorzugt aus Kunststoff ist, und die unabhängig hiervon ähnlich wie bekannte Dübel mit einem Schlitz versehen sein kann, kann beispielsweise durch eine konische oder anders geeignete Ausbildung der Verriegelungsschraube aufgeweitet und fixiert werden. Ebenso ist es möglich, diese Lagefixierung der Aufnahmeeinrichtung mit der Verriegelungsschraube durch eine zum Schaft des Marknagels bspw. koaxial und/oder im Wesentlichen parallel angeordnete Klemmschraube zu erreichen. Eine solche Klemmschraube kann dabei auch bspw. als ein Passstift oder eine verriegelbare Einrichtung oder jede andere Einrichtung ausgestaltet sein bzw. durch diese ersetzt sein, welche eine Druck- oder Zugwirkung auf die Aufnahmeeinrichtung ausüben zu deren Inmobilisierung im Raum oder in wenigstens einer Ebene kann. Falls mehrere Aufnahmeeinrichtungen im Marknagel vorgesehen sind, können diese fixiert, insbesondere positions-, und/oder winkel- und/oder lagefixiert, werden, indem bspw. zwischen den einzelnen Aufnahmeeinrichtungen Zwischenkörper vorgesehen sind, wobei dann die einzelnen Aufnahmeeinrichtungen durch eine Einrichtung, z. B. durch eine Klemmschraube, fixiert werden. Auch Kombinationen der zuvor genannten und weiterer, dem Fachmann bekannten Einrichtungen der oben genannten Art zum Erzielen einer Arretierung oder Fixierung sind von der Erfindung umfasst.

Eine solche Einrichtung - wie Zwischenkörper - ist jedoch nicht unentbehrlich. Vielmehr können die Aufnahmeeinrichtungen alternativ auch gegeneinander verblockt werden bei direktem Kontakt der Aufnahmeeinrichtungen miteinander.

Die Zwischenkörper können massiv oder mit wenigstens einem Hohlraum ausgestaltet sein. Durch die Hohlräume kann sich eine größere Deformität der Zwischenkörper ergeben, was wiederum die Reibung und somit die Winkelstabilität erhöht.

Die Zwischenkörper können so ausgeführt sein, dass sie ähnlich einer Verzahnung eine formschlüssige Verbindung mit der Aufnahmeeinrichtung eingehen können, um so bei der Verblockung einen erhöhten Reibwiderstand leisten zu können und damit die Winkelstabilität zu erhöhen.

Der erfindungsgemäße Marknagel kann in einem modularen Aufbau derart ausgestaltet sein, dass der Marknagel eine koaxiale Bohrung oder Öffnung aufweist, in welche ein Einsatz mit wenigstens einer Aufnahmeeinrichtung oder eine zusammenhängende Einheit von Aufnahmeeinrichtungen und Zwischenkörpern eingebracht werden kann. Diese Ausgestaltung erlaubt vorteilhaft, dass vor dem Einbringen des Einsatzes die Öffnung für eine Kanülierung nutzbar ist mit den für den Fachmann im Zusammenhang mit der Kanülierung bekannten Vorteilen.

Aufnahmeeinrichtungen können dabei anstelle mit Verriegelungsschrauben auch mit Platzhalten besetzt werden. Dies hat den Vorteil, dass eine nicht mit einer Verriegelungsschraube belegte Aufnahmeeinrichtung mittels des Platzhalters einen gewünschten Kraftfluss durch sie hindurch erlaubt, wenn nach Abschluss des Einbringens des Marknagels in den Knochen die axiale Verspannung für eine winkelstabile Sicherung genutzt wird.

Es wird darauf hingewiesen, dass der erfindungsgemäße Marknagel zusammen mit einer oder mehreren Verriegelungsschrauben verwendet werden kann. Daher ist die Verwendung der Begriffe "Verriegelungsschraube" und "Verriegelungsschrauben" in der vorliegenden Beschreibung austauschbar: Wo von "Verriegelungsschraube" die Rede ist, sind auch "Verriegelungsschrauben" zu verstehen und umgekehrt, wo immer dies technisch möglich ist. Dabei steht der Begriff "Verriegelungsschraube" für eine Verriegelungseinrichtung allgemein.

Daher kann die Verriegelungsschraube ferner als Verriegelungsstift ausgestaltet sein. Ein Gewinde ist erfindungsgemäß nicht erforderlich. Das Vorsehen einer Passung oder sonstigen Einrichtung zum Erhöhen der Reibung zwischen Aufnahmeeinrichtung und Verriegelungsschraube kann vorteilhaft ein mit der Zeit erfolgendes, unerwünschtes Heraustreten der Verriegelungsschraube entlang ihrer Längsrichtung aus der Aufnahmeeinrichtung verhindern. Diese Einrichtungen zum Erhöhen der Reibung können dabei über lediglich einem Abschnitt (bzw. entlang des Abschnitts) der Aufnahmeeinrichtung und/ oder der Verriegelungsschraube oder deren gesamte Erstreckung vorgesehen sein.

Unter dem "Unterbinden" wird in manchen beispielhaften Ausführungsformen des erfindungsgemäßen Marknagels ein Beenden der Ausrichtbarkeit der in die Aufnahmeeinrichtung eingeführten Verriegelungseinrichtung, z. B. einer Verriegelungsschraube, im Raum, wenigstens in zumindest einer Raumrichtung oder einer Drehrichtung, verstanden. Dies kann durch ein Blockieren, ein Arretieren, ein Aushärten, ein Verriegeln oder dergleichen der Aufnahmeeinrichtung geschehen. Hierzu kann eine Einrichtung zum Unterbinden wie einer oben beschriebenen Klemmschraube vorgesehen sein. Mit Unterbinden kann jedoch auch eine Wirkung oder Eigenschaft gemeint sein, die einer der Strukturen innewohnt. Als Beispiel wird hier die Aushärtbarkeit einer mittels Zement ausgestalteten Aufnahmeinrichtung genannt.

Ein weiteres Beispiel, wie die Ausrichtbarkeit der Aufnahmeeinrichtung unterbunden werden kann, ist das Vorsehen einer Einrichtung zur Erhöhung der Reibung zwischen Verriegelungseinrichtung und Knochennagel, wobei ein Einbringen der Verriegelungseinrichtung (bspw. durch Schrauben, Einschlagen, Einschieben oder dergleichen) in die Aufnahmeeinrichtung die Ausrichtbarkeit der Aufnahmeeinrichtung zunehmend oder schlagartig beendet. Dies kann durch ein Aufspreizen der Aufnahmeeinrichtung mittels des bspw. konisch zulaufenden Schafts der Verriegelungseinrichtung bewerkstelligt werden. Durch ein solches Aufspreizen oder auch nur eine entsprechende Druckerhöhung mit Verformung einer äußeren Kontur der Aufnahmeinrichtung oder wenigstens Erhöhen des Drucks der Aufnahmeeinrichtung auf ihre Umgebung kann die Aufnahmeeinrichtung gegen eine weitere Struktur des Marknagels derart gedrückt werden, dass die Aufnahmestruktur gegenüber dem Marknagel in ihrer Bewegungsfreiheit und insbesondere in ihrer Ausrichtbarkeit blockiert ist. Sie kann also allein durch das Eintreiben der Verriegelungseinrichtung in sie hinein arretiert werden.

Die Verriegelungseinrichtung (bspw. in Stift- oder Schraubenform) kann dabei derart ausgestaltet sein, dass erst das Einbringen ihres zuletzt in die Aufnahmeeinrichtung einzubringenden Abschnitts die Blockierung bewirkt. Es kann die Verriegelungseinrichtung für eine solche Blockierung vorbereitet sein. Die Aufnahmeeinrichtung kann ebenfalls entsprechend ausgestaltet sein. Zudem können sowohl die Verriegelungseinrichtung als auch die Aufnahmeeinrichtung zum Erzielen der Blockierung bzw. Verrieglung ausgestaltet sein.

Alle erfindungsgemäßen Gegenstände können aus einer Typ II anodisierten Titanlegierung (Ti6A14V) für verbesserte biomechanische und biomedizinische Leistungen gefertigt sein.

Die Erfindung wird im Folgenden anhand der Zeichnung näher erläutert. In dieser gilt:
- Fig. 1: zeigt einen Marknagel gemäß der Erfindung mit einer in dem Schaft des Marknagels verschwenkbar angeordneten Hülse als Beispiel einer Aufnahmeeinrichtung, die eine Verriegelungsschraube zum Fixieren des Marknagels aufnimmt;
- Fig. 2: zeigt eine schematische Darstellung einer Verriegelungsschraube und einer Hülse zur Verriegelung des Marknagels in einem Knochen;
- Fig. 3: zeigt einen in den Markraum eines Knochens implantierten Marknagel mit einer Klemmschraube zur lagestabilen Fixierung des Marknagels; und
- Fig. 4: zeigt einen Querschnitt durch einen Marknagel mit zwei jeweils zur Aufnahme einer Verriegelungsschraube ausgebildeten Aufnahmeeinrichtungen, die miteinander durch einen Zwischenkörper und eine Klemmschraube fixiert sind.

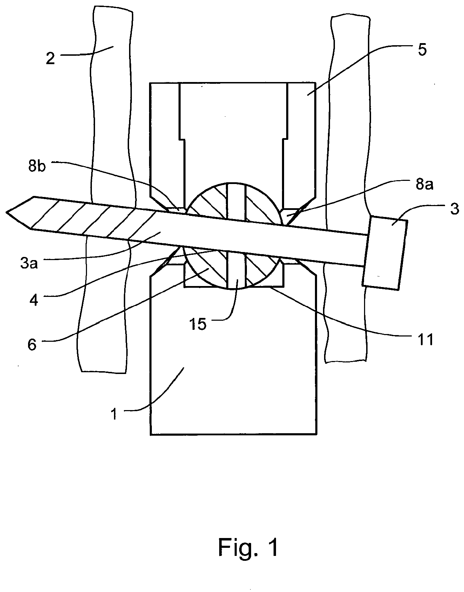

In **Fig. 1** ist ein Marknagel 1 dargestellt, der in den Markraum eines Knochens 2 implantiert ist. In den Marknagel 1 ist im Bereich, in dem eine Verriegelungsschraube 3 platziert werden soll, in einem Schaft 5 des Marknagels 1 eine kugelförmige Hülse 6 als Beispiels einer Aufnahmeeinrichtung eingebracht, die zur Aufnahme der Verriegelungsschraube 3 eine Bohrung 4 aufweist. Der Schaft 5 weist im Bereich der Hülse 6 zwei gegenüber liegende Durchbrüche 8a, 8b auf, durch welche die Verriegelungsschraube 3 hindurch geführt werden kann, wobei der Abschnitt 3a in der Hülse 6 zum Liegen kommt. Die Hülse 6 liegt auf einer Sitzfläche 11 im Marknagel 1 auf und ist frei drehbar im Schaft 5 gelagert. Hierdurch kann die Verriegelungsschraube 3 während der Operation in einem durch die Durchbrüche 8a, 8b im Schaft 5 begrenzten Winkelbereich in beliebiger Neigung durch die Bohrung 4 im Marknagel 1 hindurch getrieben werden. Auch werden leichte Fehlstellungen der Hülse 6 oder der Verriegelungsschraube 3 kompensiert, sodass die Verriegelungsschraube 3 stets in die Bohrung 4 der Hülse 6 eingreift.

Durch diese Winkelflexibilität bzw. -ausrichtbarkeit beim Einbringen der Verriegelungsschraube 3 besteht die Möglichkeit, den Marknagel 1 während der Implantation an die individuelle anatomische beziehungsweise pathologische Situation des Patienten optimal anzupassen.

Die Verriegelung der Hülse 6 in dem Schaft 5 kann auf unterschiedliche Weise erfolgen.

Die Hülse 6 des Marknagels 1 weist als Beispiel einer Aufnahmeeinrichtung 6 eine durch sie hindurchreichende Führungsdrahtöffnung 15 zum Aufnehmen eines in den Figuren nicht gezeigten Führungsdrahts auf.

Eine oder mehrere solcher Führungsdrahtöffnungen 15 können auch bei jeder der im Folgenden diskutierten und in den Figuren gezeigten Aufnahmeeinrichtungen oder Hülsen, aber auch in Zwischen- oder Stützkörpern 13 vorhanden sein, wie in den folgenden Figuren gezeigt.

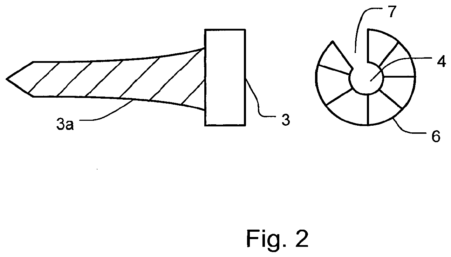

Ein Beispiel einer erfindungsgemäßen Aufnahmeeinrichtung mit Verriegelungsschraube zeigt die **Fig. 2****.** Die Verriegelungsschraube 3 weist hierbei einen teilweise oder vollständig konisch ausgebildeten Schraubenschaft 3a auf; die Hülse 6 mit ihrer Öffnung 4 ist ähnlich einem Dübel mit einem Schlitz 7 versehen. Die Hülse 6 wird dann beim Eindrehen der Verriegelungsschraube 3 aufgeweitet und im Schaft 5 des Marknagels 1 verspreizt. Die Hülse legt sich dann an entsprechende Anschläge, zum Beispiel die Ränder der Durchbrüche 8a und 8b an. Durch diese kraftschlüssige Fixierung der kugelförmigen Hülse 6 in Schaft 5 wird die Verriegelungsschraube 3 dauerhaft in der gewählten Lage winkelstabil verriegelt.

Zur Erzielung einer optimalen Fixierung kann die Hülse 6 aus einem entsprechend spreizbaren Material, zum Beispiel einem für diese Anwendung geeigneten Kunststoff bestehen bzw. ein solches aufweisen.

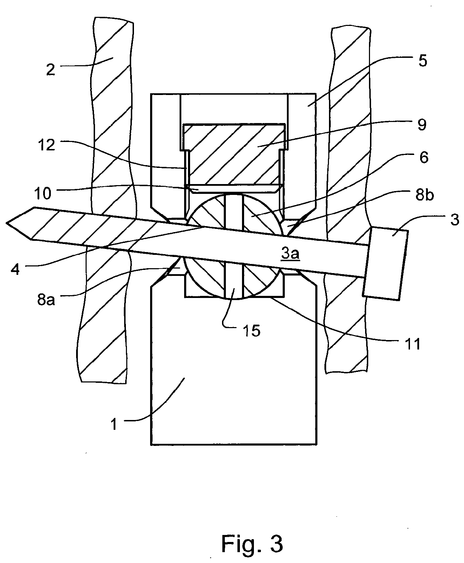

In **Fig. 3** ist eine andere Möglichkeit der winkelstabilen Arretierung der Hülse 6 mit der Verriegelungsschraube 3 gezeigt. Die Ausführung des Marknagels 1, der Hülse 6 und der Verriegelungsschraube 3 ist ähnlich wie in Fig. 1 dargestellt. Die kugelförmige Hülse 6 kann hierbei wiederum aus einem entsprechenden Kunststoff bestehen. Die Kugel bzw. Hülse 6 liegt im Schaft 5 des Marknagels auf einer Sitzfläche 11 auf.

Oberhalb der Kugel bzw. Hülse 6 ist der Schaft 5 mit einem Gewinde 12 versehen, in das koaxial zum Schaft eine Klemmschraube 9 eingesetzt ist, die auf der Oberseite der Hülse 6 aufliegt. Durch Einschrauben der Klemmschraube 9 in das Gewinde 12 wird die Hülse auf die Sitzfläche 11 gepresst und nach dem Einbringen der Verriegelungsschraube 3 lagestabil fixiert.

Zur Erhöhung der Klemmkraft und des daraus resultierenden Lösemoments der Hülse 6 im Schaft 5 kann die Klemmschraube 9 an ihrer Kontaktfläche 10 mit der Hülse 6 entsprechend geometrisch an die Form der Hülse angepasst werden, was hier nicht dargestellt ist. Hierdurch kann die Haftreibung zwischen Klemmschraube und Hülse erhöht werden.

Zusätzlich kann die Kontaktfläche der Klemmschraube beispielsweise durch Rändeln oder das Aufbringen reibungsteigernder Beschichtungen aufgeraut werden. Eine andere Möglichkeit wäre, die Klemmschraube 9 mit einem spitz zulaufenden Ende zu versehen, welches in die Hülse 6 eindringt und diese somit zusätzlich formschlüssig fixiert.

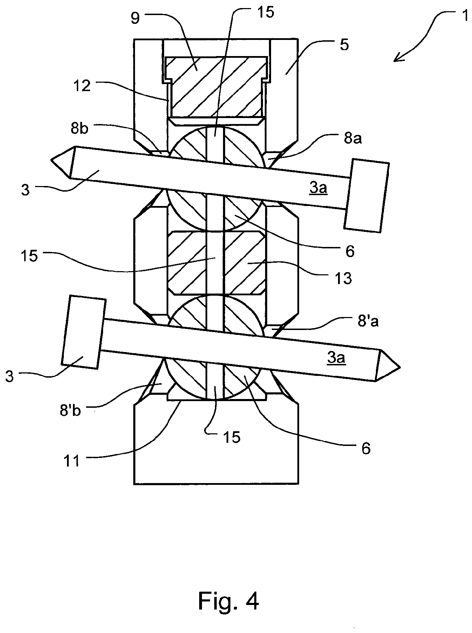

In **Fig. 4** ist ein Marknagel 1 mit einem Schaft 5 dargestellt, der mehrere, in diesem Falle zwei Durchbrüche 8a, 8b und 8'a, 8'b aufweist, wobei in diesen Bereichen jeweils eine Hülse 6 eingesetzt ist, in die jeweils eine Verriegelungsschraube 3 hindurch getrieben werden kann.

Zur Verriegelung der beiden Hülsen 6 ist wiederum eine Klemmschraube 9 entsprechend Fig. 3 vorgesehen, die auf der oberen Hülse 6 anliegt. Zwischen den beiden Hülsen 6 ist ein blockförmiger Zwischenkörper 13 vorgesehen, der an beiden Hülsen 6 anliegt. Durch Festziehen der Klemmschraube 9 werden dann beide Hülsen 6 unter Zwischenschaltung des Zwischenkörpers 13 lagestabil arretiert. Auch der Zwischenkörper 13 weist (wenigstens) eine Führungsdrahtöffnung 15 auf.

## Patentansprüche

1. Marknagel (1), zum Einbringen in einen Röhrenknochen, mit einem Schaft (5) mit einer Längsachse, wobei der Schaft wenigstens eine Durchgangsöffnung (8) zum Einbringen wenigstens einer Verriegelungseinrichtung in ein Inneres des Marknagels (1) und/oder zum Hindurchführen durch den Marknagel (1), aufweist;
wobei der Marknagel (1) wenigstens eine Aufnahmeeinrichtung (6) zum Aufnehmen wenigstens eines Abschnitts (3a) der Verriegelungseinrichtung aufweist;
wobei die wenigstens eine Aufnahmeeinrichtung (6) eine Hülse in Form einer Walze mit einer Walzenachse, welche senkrecht zu einer Längsachse des Schafts (5) des Marknagels (1) steht, ist, oder eine kugelförmige Hülse ist, oder eine Hülse von elliptischem oder elliptoidem Durchmesser ist;
wobei die wenigstens eine Aufnahmeeinrichtung (6) in wenigstens einer ihrer Raumachsen in Bezug zur Längsachse des Schafts (5) bewegbar oder ausrichtbar innerhalb des Schafts (5) oder der Durchgangsöffnung (8) angeordnet ist, wobei die Bewegung eine Bewegung relativ zur Längsachse des Schafts ist;
wobei der Marknagel (1) wenigstens eine Einrichtung zum Unterbinden der Ausrichtbarkeit der wenigstens einen Aufnahmeeinrichtung (6) in Bezug zur Längsachse und/oder zu einer hierzu senkrechten Querachse aufweist; und wobei die wenigstens eine Aufnahmeeinrichtung (6) eine Öffnung oder Bohrung zur Aufnahme der wenigstens einen Verriegelungseinrichtung aufweist;
**dadurch gekennzeichnet, dass**
die wenigstens eine Aufnahmeeinrichtung (6) wenigstens eine durch sie hindurchreichende
Führungsdrahtöffnung (15) zum Aufnehmen eines Führungsdrahts aufweist, und dass
die wenigstens eine Führungsdrahtöffnung (15) in manchen ihrer Abschnitte durch die Öffnung oder Bohrung der wenigstens einen Aufnahmeeinrichtung (6) zur Aufnahme wenigstens einer Verriegelungseinrichtung hindurch geht, in anderen Abschnitten der Führungsdrahtöffnung (15) hingegen nicht.

2. Marknagel nach Anspruch 1, **dadurch gekennzeichnet, dass** die Aufnahmeeinrichtung (6) eine Hülse (6) mit einer Hülsenachse senkrecht zu der Längsachse des Schafts (5) des Verriegelungsmarknagels (1) ist.

3. Marknagel nach Anspruch 1, **dadurch gekennzeichnet, dass** die Aufnahmeeinrichtung (6) in zumindest einem Abschnitt hiervon kugelförmig ist und in mehr als einer Raumachse, insbesondere in allen Raumachsen, frei schwenkbar oder drehbar in dem Schaft (5) angeordnet, insbesondere gelagert ist.

4. Marknagel nach einem der vorhergehenden Ansprüche, **gekennzeichnet durch** eine Einrichtung zum kraftschlüssigen und/oder formschlüssigen Arretieren der Aufnahmeeinrichtung (6) durch Kraftschluss unter Unterbinden der Ausrichtbarkeit.

5. Marknagel nach einem der vorhergehenden Ansprüche, **dadurch gekennzeichnet, dass** die Aufnahmeeinrichtung (6) geschlitzt oder aufweitbar ausgeführt ist, und dass die zugehörige Verriegelungsschraube (3) zur Lagefixierung zumindest in einem Bereich konisch ausgeführt ist.

6. Marknagel nach einem der vorhergehenden Ansprüche, **gekennzeichnet durch** wenigstens oder genau eine Klemmschraube (9) zum Unterbinden der Ausrichtbarkeit der wenigstens einen Aufnahmeeinrichtung (6) und/oder zur Fixierung von deren Lage.

7. Marknagel nach einem der vorhergehenden Ansprüche, **dadurch gekennzeichnet, dass** die Aufnahmeeinrichtung (6) Kunststoff aufweist oder aus Kunststoff besteht.

8. Marknagel nach einem der vorhergehenden Ansprüche, **dadurch gekennzeichnet, dass** bei mehreren aufeinander folgenden Aufnahmeeinrichtungen (6) in oder am Marknagel (1) zwischen den Aufnahmeeinrichtungen (6) jeweils an diesen anliegende Stützkörper (13) vorgesehen sind, wobei die Anordnung aus Aufnahmeeinrichtungen (6) und Stützkörpern (13) durch zumindest eine in Längsrichtung des Marknagels (1) wirkende Klemmschraube (9) zusammenpressbar und/oder blockierbar und/oder arretierbar ist.

9. Marknagel nach einem der vorhergehenden Ansprüche, **dadurch gekennzeichnet, dass** wenigstens einer der Stützkörper (13) wenigstens eine Führungsdrahtöffnung (15) aufweist.

10. Marknagel nach einem der vorhergehenden Ansprüche, **dadurch gekennzeichnet, dass** sich bei mehreren aufeinander folgenden Aufnahmeeinrichtungen (6) in oder am Marknagel (1) wenigstens zwei der Aufnahmeeinrichtungen (6) durch das Einwirken einer Klemmschraube (9) gegenseitig zusammenpressen oder blockieren oder arretieren.

## Claims

1. An intramedullary nail (1) for insertion into a tubular bone, comprising a shaft (5) with a longitudinal axis,
the shaft comprising at least one through-opening (8) for inserting at least one locking device into an interior of the intramedullary nail (1) and/or for passing through the intramedullary nail (1);
the intramedullary nail (1) comprising at least one receiving device (6) for receiving at least a portion (3a) of the locking device;
wherein the at least one receiving device (6) is a sleeve in the form of a roller having a roller axis perpendicular to a longitudinal axis of the shaft (5) of the intramedullary nail (1), or is a spherical sleeve or a sleeve of elliptical or elliptoid diameter;
wherein the at least one receiving device (6) is movably or alignably arranged within the shaft (5) or the through opening (8) in at least one of its spatial axes in respect to the longitudinal axis of the shaft (5), the movement being a movement relative to the longitudinal axis of the shaft;
wherein the intramedullary nail (1) comprises at least one device for preventing the alignment of the at least one receiving device (6) in respect to the longitudinal axis and/or in respect to a transverse axis perpendicular thereto;
and wherein the at least one receiving device (6) comprises an opening or a bore for receiving the at least one locking device;
**characterized in that**
the at least one receiving device (6) comprises at least one guide wire opening (15) extending through it for receiving a guide wire, and **in that**
the at least one guide wire opening (15) passes through the opening or the bore of the at least one receiving device (6) for receiving at least one locking device in some of its portions, but not in other portions of the guide wire opening (15).

2. The intramedullary nail according to claim 1, **characterized in that** the receiving device (6) is a sleeve (6) having a sleeve axis perpendicular to the longitudinal axis of the shaft (5) of the locking intramedullary nail (1).

3. The intramedullary nail according to claim 1, **characterized in that** the receiving device (6) is spherical in at least one portion thereof and is, in more than one spatial axis, in particular in all spatial axes, freely pivotably or rotatably arranged in particular mounted, in the shaft (5).

4. The intramedullary nail according to anyone of the preceding claims, **characterized by** a device for frictional and/or form-fit locking of the receiving device (6) by means of frictional connection while preventing alignment.

5. The intramedullary nail according to anyone of the preceding claims, **characterized in that** the receiving device (6) is split or expandable designed, and **in that** the associated locking screw (3) for fixing the position is conical in at least one region.

6. The intramedullary nail according to anyone of the preceding claims, **characterized by** at least, or precisely, one clamping screw (9) for preventing the alignment of the at least one receiving device (6) and/or for fixing its position.

7. The intramedullary nail according to anyone of the preceding claims, **characterized in that** the receiving device (6) comprises plastic or consists of plastic.

8. The intramedullary nail according to anyone of the preceding claims, **characterized in that**, in the case of several successive receiving devices (6), supporting bodies (13) are provided in or on the intramedullary nail (1), between and adjacent to the receiving devices (6) respectively, the arrangement of receiving devices (6) and supporting bodies (13) being compressible and/or blockable and/or lockable by at least one clamping screw (9) acting in the longitudinal direction of the intramedullary nail (1).

9. The intramedullary nail according to anyone of the preceding claims, **characterized in that** at least one of the supporting bodies (13) comprises at least one guide wire opening (15) .

10. The intramedullary nail according to anyone of the preceding claims, **characterized in that**, in the case of several successive receiving devices (6) in or on the intramedullary nail (1), at least two of the receiving devices (6) mutually compress or block or lock each other by the action of a clamping screw (9).

## Revendications

1. Un clou intramédullaire (1) destiné à être inséré dans un os tubulaire, comprenant une tige (5) avec un axe longitudinal, la tige comprenant au moins un orifice de passage (8) pour l'insertion d'au moins un dispositif de verrouillage à l'intérieur du clou intramédullaire (1) et/ou pour le passage au travers du clou intramédullaire (1);
le clou intramédullaire (1) comprenant au moins un dispositif de réception (6) pour recevoir au moins une section (3a) du dispositif de verrouillage;
où au moins ledit dispositif de réception (6) est un manchon sous forme d'un rouleau ayant un axe de rouleau perpendiculaire à un axe longitudinal de la tige (5) du clou intramédullaire (1), ou est un manchon sphérique ou un manchon de diamètre elliptique ou elliptoïdal;
où au moins ledit dispositif de réception (6) est agencé de façon mobile ou alignable à l'intérieur de la tige (5) ou de l'orifice de passage (8) pour au moins un de ses axes spatiaux par rapport à l'axe longitudinal de la tige (5), le mouvement étant un mouvement relatif à l'axe longitudinal de la tige;
où le clou intramédullaire (1) comprend au moins un dispositif pour empêcher l'alignement d'au moins ledit dispositif de réception (6) par rapport à l'axe longitudinal et/ou par rapport à un axe transversal perpendiculaire à celui-ci;
et où au moins ledit dispositif de réception (6) comprend un orifice ou un alésage pour la réception d'au moins ledit dispositif de verrouillage;
**caractérisé en ce que**
au moins ledit dispositif de réception (6) comprend au moins une ouverture pour guidage de fil (15) s'étendant au travers de celui-ci pour la réception d'un fil de guidage, et **en ce que**
au moins ladite ouverture pour guidage de fil (15) passe au travers de l'orifice ou de l'alésage d'au moins dudit dispositif de réception (6) pour la réception d'au moins un dispositif de verrouillage dans certaines de ses sections, mais pas dans d'autres sections de l'ouverture pour guidage de fil (15).

2. Le clou intramédullaire selon la première revendication, **caractérisé en ce que** le dispositif de réception (6) est un manchon (6) ayant un axe de manchon perpendiculaire à l'axe longitudinal de la tige (5) du clou intramédullaire de verrouillage (1).

3. Le clou intramédullaire selon la première revendication, **caractérisé en ce que** le dispositif de réception (6) est sphérique dans au moins une de ses sections et est agencé, voire monté, de façon librement pivotable ou rotative dans la tige (5) pour plus d'un axe spatial, voire pour tous les axes spatiaux.

4. Le clou intramédullaire selon l'une quelconque des revendications précédentes, **caractérisé par** un dispositif permettant un verrouillage en force et/ou par complémentarité de forme du dispositif de réception (6) par adhérence tout en empêchant l'alignement.

5. Le clou intramédullaire selon l'une quelconque des revendications précédentes, **caractérisé en ce que** le dispositif de réception (6) est fendu ou extensible, et **en ce que** la vis de verrouillage (3) associée permettant de fixer la position est conique au moins dans une région.

6. Le clou intramédullaire selon l'une quelconque des revendications précédentes, **caractérisé par** au moins ou précisément une vis de serrage (9) pour empêcher l'alignement d'au moins un dispositif de réception (6) et/ou pour fixer sa position.

7. Le clou intramédullaire selon l'une quelconque des revendications précédentes, **caractérisé en ce que** le dispositif de réception (6) comprend du plastique ou est constitué de plastique.

8. Le clou intramédullaire selon l'une quelconque des revendications précédentes, **caractérisé en ce que**, dans le cas de plusieurs dispositifs de réception (6) successifs, des corps de support (13) sont prévus dans ou sur le clou intramédullaire (1) entre les dispositifs de réception (6) et adjacents à ceux-ci, respectivement, l'agencement des dispositifs de réception (6) et des corps de support (13) pouvant être compressible et/ou blocable et/ou verrouillable au moyen d'au moins une vis de serrage (9) agissant dans une direction longitudinale du clou intramédullaire (1).

9. Le clou intramédullaire selon l'une quelconque des revendications précédentes, **caractérisé en ce qu'**au moins l'un de corps de support (13) comprend au moins une ouverture de guidage de fil (15).

10. Le clou intramédullaire selon l'une quelconque des revendications précédentes, **caractérisé en ce que**, dans le cas de plusieurs dispositifs de réception (6) successifs dans ou sur le clou intramédullaire (1), au moins deux des dispositifs de réception (6) se compressent ou se bloquent ou se verrouillent mutuellement par l'action d'une vis de serrage (9).
